# EUROPEAN PATENT SPECIFICATION

(11) **EP 0 797 986 B1**
(45) Date of publication and mention of the grant of the patent: **22.01.2003**
(21) Application number: 97105099.2
(22) Date of filing: 26.03.1997
(51) Int. Cl.: A61K 9/00

(54) **Veterinary compositions containing antibacterials for intramammary use**
Veterinäre Zusammensetzungen mit antibakteriellen Mitteln zur intramammären Anwendung
Compositions vétérinaires à base d'antibactériens pour l'usage intramammaire

(30) Priority: 02.04.1996 IT MI960640
(43) Date of publication of application: 01.10.1997
(73) Proprietor: FATRO S.p.A., I-40064 Ozzano Emilia (Bologna) (IT)
(72) Inventor: Bernabei, Silvia, 40064 Ozzano Emilia (BO) (IT); Corti, Piero, 53100 Siena (SI) (IT)
(74) Representative: Minoja, Fabrizio, Dr.

(56) References cited:
- GB-A- 2 273 655
- US-A- 3 679 787

## Description

The present invention relates to aqueous solutions of antibacterial agents thickened with a suitable gelifying agent, for the intramammary administration in the treatment of mastitis.

### PRIOR ART

At present, the pharmaceutical forms known for the intramammary administration in the treatment of mastitis consist of the active principles dispersed or dissolved in oily systems.

Examples of said antibacterial agents include antibiotics such as neomycin sulfate, streptomycin sulfate, dihydrostreptomycin sulfate, tetracycline soluble salts, oxytetracycline, chlortetracycline, rifamycin SV, rifaximin, penicillins such as sodium penicillin, sodium ampicillin, sodium cloxacillin, sodium dicloxacillin, mixtures of two penicillins such as sodium ampicillin and sodium cloxacillin, cephalosporin sodium salts, lincomycin hydrochloride, spectinomycin hydrochloride and sulfate, thiamphenicol glycinate acetylcysteinate.

These active principles are incorporated in the formulation at such concentrations as to exert their antibacterial activities.

Said pharmaceutical forms, used for anti-mastitis treatments during lactation, allow only a partial release of the active ingredient in the time useful to exert the pharmacological activity, thus decreasing the potency of the therapeutical action.

Moreover, the complete elimination of any active ingredient residues with milking is not assured, resulting in an economic damage to the farmer.

A significant improvement in the performances of the conventional pharmaceutical forms has been introduced by EP 222 712 in the Applicant's name, in which an oily excipient was used, but the optimization of the active ingredient bioavailability was obtained with the use of a hydrophilic surfactant. A rapid release of the active ingredient and a satisfactory therapeutical effectiveness were thereby guarantied.

US 3, 679, 787 discloses veterinary compositions containing a combination of lincomycin and spectinomycin, useful in treating diseased animals and exhibiting a synergistic activity. Suitable forms include tablets, powders, wafers, cachets, granules, pills, capsules, dispersion in edible oils, aqueous dispersions.

GB 2,273,655 discloses compositions for treating mastitis by the intramammary route, which comprise an insoluble antibiotic in aqueous suspensions. The composition may further comprise a gel seal containing a heavy metal salt.

Now it has surprisingly been found that the intramammary administration of antibiotics or chemotherapeutics in the form of a hydrogel (aqueous gel) is particularly effective and assures the rapid elimination of the active principles.

Moreover, the composition in the form of an aqueous gel showed an excellent tolerability; the reduced hystologic damages of the aqueous carrier can in fact be evidenced as a faster decrease in the cell count after the treatment.

A further advantage is the easy, economical process for the preparation of the formulation, thanks to the use of inexpensive, highly safe excipients.

### DISCLOSURE OF THE INVENTION

The formulation of the invention consists of an aqueous solution of antibiotics thickened with a suitable gelifying agent.

In the invention, antibiotics in the form of water soluble salts stable in an aqueous carrier are preferred.

Particularly suitable to this novel formulation proved to be the combination of the salts of lincomycin and spectinomycin, such as the hydrochloride salts.

The combination of the active principles, thanks to their synergism against the strain usually responsible for mammary diseases, turned out to be particularly effective in the therapy of bovine mastitis.

The lincomycin/spectinomycin combination in an aqueous gel formulation showed a rapid absorption and an improved tolerability.

The high water solubility and stability in water of lincomycin hydrochloride and of spectinomycin hydrochloride and/or sulfate makes these two antibiotics particularly suitable to the novel aqueous gel formulation.

The hydrophilic carrier (preferably water, but also alcohols and polialcohols, such as propylene glycol, polyethylene glycol and the like) is formulated as to be non-toxic, suitable to the veterinary use, compatible with the considered antibacterial agents and of such viscosity as to allow the intramammary administration at room temperature, through mono-dose devices, preferably syringes.

The formulation can contain the active principles up to a total maximum content corresponding to saturation of the aqueous solution .

The active principles can be used in any ratio (preferably lincomycin:spectinomycin 1:2) as deduced from literature, for the optimization of the therapeutical activity.

The carrier is an aqueous solution buffered to acid pH, preferably 4.5.

Suitable buffer systems are pharmaceutically acceptable acids and salts such as carboxylic acids (acetic, malonic, fumaric, maleic, succinic, lactic, citric acids) or phosphate buffers, in concentration of 0.05-0.5M.

Preferably the used buffer system consists of acetic acid/sodium acetate in a 0.05-0.5M concentration, preferably 0.1M, with a pH ranging from 3 to 5.5, preferably 4.5.

Stable, injectable pharmaceutical forms are obtained by gelification of the aqueous solution gives with the conventional gelification agents such as: alginates, variously derivatized carboxyvinyl polymers, polyvinyl alcohol, variously derivatized celluloses, polyvinylpyrrolidone, and other hydrocolloids, pectins, gelatins, preferably hydroxypropyl methylcellulose, at concentrations varying from 0.1 to 30% w/w, preferably 2.5% w/w.

The surfactant agent preferably has a high HLB, thereby significantly improving the water solubility of the active ingredient and promoting the dissolution of the pharmaceutical form in the biological matrix.

The tested compounds belong to the series of the surfactants, preferably hydrophilic or anyhow with HLB values above 8, preferably 15, polyethylene glycol 660-12-hydroxystearate at concentrations of 0.1 to 5%, preferably 0.5% w/w, being most preferred.

A unitary dose of the pharmaceutical form contains 1 to 20 ml of the formulated composition, preferably 2 to 10 ml, most preferably 5 ml.

The formulation can also contain other additives, for example preservatives, antioxidants in amounts of 0.05-1.0%.

The antibacterial preservatives are selected from the group of the compounds water soluble, at .a 0.1-2% concentration, preferably 1% benzyl alcohol.

Among all the possible antioxidant preservatives, sodium metabisulfite at concentrations of 0.05 to 1%, preferably 0.3%, is preferred.

The following example will illustrate the process for the preparation of the formulation of the invention.

### EXAMPLE

75 ml of water are added under stirring with 0.1 g of sodium metabisulfite, 0.3 g of Tween 80®, 1 g of benzyl alcohol and 1.2 g of sodium dihydrogen phosphate.

22.5 g of spectinomycin hydrochloride and 7.5 g of lincomycin hydrochloride are added.

Stirring is continued until complete dissolution, adjusting pH between 4 and 5.

The solution is sterile filtered and placed into a turbodiffuser.

2.5 g of hydroxypropyl methylcellulose are added under mechanical stirring for 15-30'.

The resulting gel is ready for distribution in syringes or tubes for the intramammary administration.

The compositions A, B, C reported in the following Table have been prepared with a process similar to that of the example.

**TABLE**

| % Compositions | (A) | (B) | (c) |
|---|---|---|---|
| Spectinomycin hydrochlor. | 22.5 | 15 | 15 |
| Lincomycin hydrochloride | 7.5 | 5 | 5 |
| Sodium metabisulfite | 0.3 | 0.3 | |
| Benzyl alcohol | 1 | 2 | |
| Poloxamer 407® | 20 | | |
| Sodium formaldehyde sulf. | 0.2 | | |
| Polyoxyethylene 600-12 hydroxystearate | | 1 | |
| Sodium methyl p.hydroxy. | | | 0.15 |
| Propylene glycol | | | 30 |
| Polyoxyethylene cetyl or stearyl alcohols | | | 1.5 |
| 0.1M Citric acid | 1.92 | | |
| Acid acetic 0.1M | | 0.6 | 0.6 |
| Sodium acetate 0.1M | | 0.82 | 0.82 |
| Water q.s. to | 100 ml | 100 ml | 100 ml |
| Sodium hydroxide q.s. to pH | 4 | 4.5 | 5 |

## Claims

1. The use of an aqueous gel containing water-soluble antibiotics which are stable in an aqueous solution for the preparation of a veterinary composition for intramammary administration to be used in the prophylaxis and therapy of mastitis.

2. The use according to claim 1, wherein said antibiotics are salts of lincomycin and spectinomycin.

3. The use according to claims 1-2, wherein the aqueous gel comprises, as the gelification agents, alginates, variously derivatized carboxyvinyl polymers, polyvinyl alcohol, variously derivatized celluloses, polyvinylpyrrolidone and other hydrocolloids, pectins, gelatins.

4. The use according to claim 3, wherein said gelification agents are present in concentrations from 0.1 to 30% w/w.

5. The use according to claims 3-4, wherein the veterinary composition comprises active ingredients up to saturated solution and 0.1-5% surfactants, 0.1-30% gelifying agents, 0.05-1% antioxidants, 0.05-0.5 M buffers, 0.1-2% antimicrobial preservatives.

6. The use according to any preceding claim, for the therapy of mastitis during lactation.

## Patentansprüche

1. Verwendung eines wässrigen Gels, das in wässriger Lösung stabile wasserlösliche Antibiotika enthält, zur Herstellung eines Tierarzneimittels zur intramammären Verabreichung bei der Prophylaxe und Therapie von Mastitis.

2. Verbindung nach Anspruch 1, **dadurch gekennzeichnet, dass** die Antibiotika Salze von Lincomycin und Spectinomycin sind.

3. Verwendung nach den Ansprüchen 1-2, **dadurch gekennzeichnet, dass** das wässrige Gel als Gelierungsmittel Alginate, verschieden derivatisierte Carboxyvinylpolymere, Polyvinylalkohol, verschieden derivatisierte Cellulosen, Polyvinylpyrrolidon und andere Hydrokolloide, Pektine, Gelatine umfasst.

4. Verwendung nach Anspruch 3, **dadurch gekennzeichnet, dass** die Gelierungsmittel in Konzentrationen von 0,1 bis 30% Gew./Gew. vorhanden sind.

5. Verwendung nach den Ansprüchen 3-4, **dadurch gekennzeichnet, dass** das Tierarzneimittel die Wirkstoffe bis zu einer gesättigten Lösung und 0,1-5% oberflächenaktive Mittel, 0;1-30% Gelierungsmittel, 0,05-1% Antioxidantia, 0,05-0,5 M Puffer, 0,1-2% antimikrobielle Konservierungsmittel enthält.

6. Verwendung nach einem der vorstehenden Ansprüche zur Therapie der Mastitis während der Laktation.

## Revendications

1. Utilisation d'un gel aqueux contenant des antibiotiques solubles dans l'eau qui sont stables dans une solution aqueuse pour la préparation d'une composition vétérinaire destinée à une administration intramammaire à utiliser dans la prophylaxie et la thérapie de la mastite.

2. Utilisation selon la revendication 1, dans laquelle lesdits antibiotiques sont des sels de lincomycine et de spectinomycine.

3. Utilisation selon les revendications 1 et 2, dans laquelle le gel aqueux comprend, en tant qu'agents de gélification, des alginates, des polymères carboxyvinyliques diversement transformés en dérivés, de l'alcool polyvinylique, des celluloses diversement transformés en dérivés, de la polyvinylpyrrolidone et d'autres hydrocolloïdes, des pectines, des gélatines.

4. Utilisation selon la revendication 3, dans laquelle lesdits agents de gélification sont présents dans des concentrations de 0,1 à 30% p/p.

5. Utilisation selon les revendications 3 et 4, dans laquelle la composition vétérinaire comprend des ingrédients actifs jusqu'à une solution saturée et de 0,1 à 5% de surfactants, de 0,1 à 30% d'agents de gélification, de 0,05 à 1% d'antioxydants, de solutions tampons 0,05 à 0,5 M, de 0,1 à 2% de conservateurs antimicrobiens.

6. Utilisation selon l'une quelconque des revendications précédentes, pour la thérapie de la mastite. durant la lactation.
